# EUROPEAN PATENT APPLICATION

(11) **EP 4 446 306 A1**
(43) Date of publication of application: **16.10.2024**
(21) Application number: 22903157.0
(22) Date of filing: 17.11.2022
(51) Int. Cl.: C07C 69/017, C07C 271/44, C07C 219/04, C07C 229/12, A61P 1/08, A61K 31/05

(54) **CANNABIDIOL DERIVATIVE, PREPARATION METHOD THEREFOR AND APPLICATION THEREOF**

(30) Priority: 10.12.2021 CN 202111506679
(71) Applicant: Deyi Pharmaceutical Ltd., Kunming, Yunnan 650100 (CN)
(72) Inventor: WANG, Shubin, Kunming, Yunnan 650100 (CN); DU, Yesong, Kunming, Yunnan 650100 (CN); ZHANG, Pingping, Kunming, Yunnan 650100 (CN)
(74) Representative: HGF
(86) International application number: PCT/CN2022/132587
(87) International publication number: WO 2023/103734

(57) **Abstract**

Disclosed in the present invention are a cannabidiol derivative, a preparation method therefor and an application thereof, particularly an application in preventing and treating nervous system diseases (such as Parkinson's disease). The cannabidiol derivative is obtained by screening from a series of synthetic derivatives. An animal testing result shows that the cannabidiol derivative can reduce the balance beam score of a Parkinson's model animal and improve the dopamine level and the tyrosine hydroxylase (TH) cell positive rate in substantia nigra (SubN), can be used for drug development and research of various diseases such as epilepsy and Parkinson's disease, and has better application value.

## Description

### TECHNICAL FIELD

The present invention relates to the technical field of chemical pharmaceuticals, and in particular to a cannabidiol derivative, a preparation method therefor, and use thereof, particularly use thereof in the prevention and treatment of a nervous system disease (e.g., Parkinson's disease).

### BACKGROUND

Cannabidiol (CBD) is a nontoxic phenolic substance with high added value extracted from flowers and leaves of *Cannabis saliva*, which can be used in medicine, cosmetics, and health food. CBD, with the chemical name of 2-[(1*R*,6*R*)-3-methyl-6-(1-methylvinyl)-2-cyclohexen-1-yl]-5-pentyl-1,3-benzenediol and CAS registry number of 13956-29-1, has a chemical structure shown as follows:

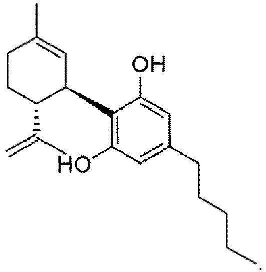

The modern medical research has shown that CBD has significant efficacy in epilepsy resistance, stress resistance, inflammation resistance, and the like. CBD has been approved by the U.S. Food and Drug Administration for the treatment of epilepsy, and can also be used as a dietary supplement for improving mood and resisting anxiety. However, cannabinoids have poor water solubility and low oral bioavailability, and thus are not favorable for development and utilization of drugs. Therefore, it is necessary to provide a new cannabidiol derivative, a preparation method therefor and use thereof, so as to provide better water solubility while ensuring high purity of CBD, thereby further improving the bioavailability of CBD and expanding the use of CBD in the field of pharmaceuticals.

### SUMMARY

In order to overcome the defects of the prior art, the present invention provides a cannabidiol derivative, a preparation method therefor, and use thereof, particularly use thereof in the prevention and treatment of a nervous system disease (e.g., Parkinson's disease).

In a first aspect, the present invention provides a compound having the following structure: 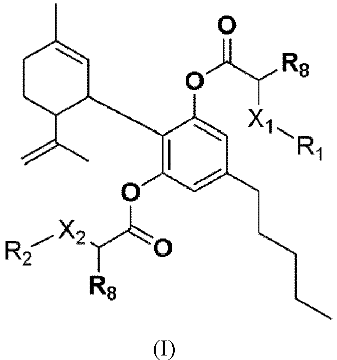 wherein,
R₈ is selected from: -NH₂ and -H; preferably, R₈ is -NH₂;
X₁ and X₂ are independently selected from: a single bond, alkylene (e.g., C₁₋₁₅ alkylene, C₁₋₁₀ alkylene, or C₁₋₅ alkylene), alkylene substituted with one or more substituents, alkylene spaced by one or more -C(=O)O-, alkylene spaced by one or more -OC(=O)-, alkylene spaced by one or more alkylimino, alkylene spaced by one or more - OC(=O)O-, and alkylene spaced by a combination of the above spacer groups; the substituent is selected from: alkyl, hydroxy, halogen, aryl, cycloalkyl, thioalkoxy, nitro, cyano, amino, heteroaryl, and heterocycloalkyl, wherein H on the C atoms can be substituted with halogen, -OC₀₋₁₀ alkyl, linear/branched C₁₋₁₀ alkyl, nitro, cyano, or amino;
further, X₁ and X₂ are independently selected from: C₁₋₅ alkylene and alkylene substituted with one or more substituents selected from: alkyl, hydroxy, halogen, aryl, cycloalkyl, thioalkoxy, nitro, cyano, amino, heteroaryl, and heterocycloalkyl, wherein H on the C atoms can be substituted with halogen, -OC₀₋₁₀ alkyl, linear/branched C₁₋₁₀ alkyl, nitro, cyano, or amino;
further, X₁ and X₂ are independently selected from: -CH₂-, -CH₂CH₂-, -(CH₂)₂CH₂-, -(CH₂)₃CH₂-, - CH₂CH(CH₃)₂-, -(CH₂)₄CH₂-, -CH(CH₃)CH₂CH₂-, and -CH(OH)CH₂-;
preferably, X₁ and X₂ are both -(CH₂)₃CH₂-,
R₁ and R₂ are independently selected from: -H, 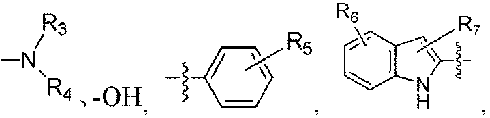 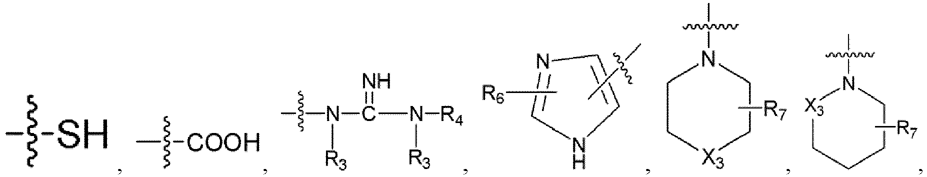 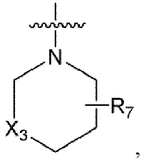 and alkoxy, wherein, X₃ is selected from N, O, and S; preferably, R₁ and R₂ are independently selected from: 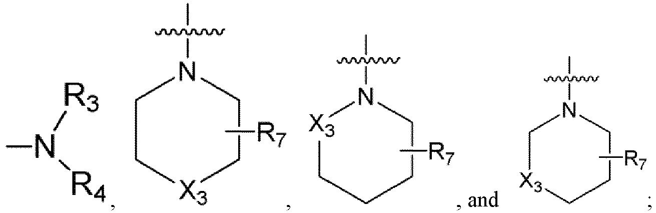
R₃ and R₄ are each independently selected from: -H, halogen, alkyl, cycloalkyl, aryl, heteroaryl, and heterocycloalkyl; preferably, R₃ and R₄ are both -H;
R₅, R₆, and R₇ are each independently selected from: -H, -OH, halogen, -NO₂, -CN, alkyl, and cycloalkyl; preferably, R₅ is selected from: -H or -OH, and R₆ and R₇ are both -H.

Further, R₁ and R₂ are independently selected from: -NH₂, -OH, 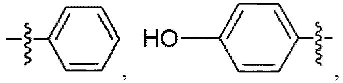 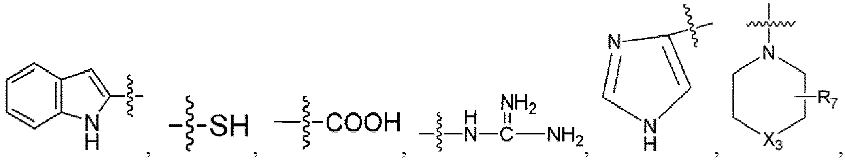 and alkoxy, wherein, X₃ is selected from N, O, and S, preferably, X₃ is O; preferably, R₁ and R₂ are independently selected from: -NH₂ and 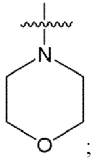 more preferably, R₁ and R₂ are both -NH₂.

In one embodiment of the present invention, R₁ and R₂ are both -NH₂.

Optionally, general formula (I) may be substituted with 1 or more D atoms.

In one embodiment of the present invention, the compound described above has the following structure:

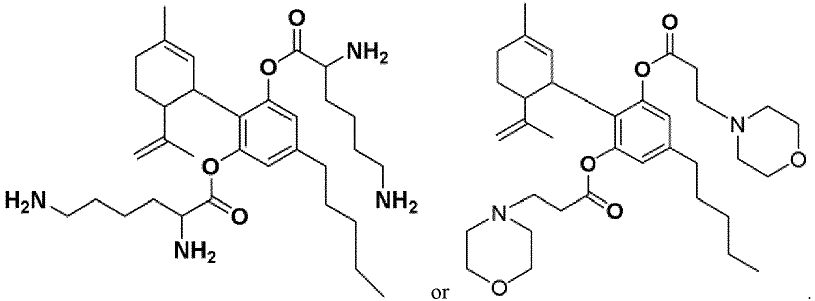

In a second aspect of the present invention, provided is a stereoisomer of the compound according to the first aspect, which has the following structure: 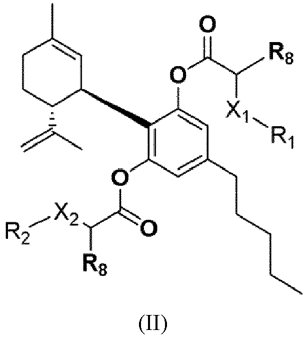 wherein, X₁, X₂, R₁, R₂, R₃, R₄, R₅, R₆, R₇, and R₈ have corresponding definitions as described in the first aspect of the present invention.

Further, the stereoisomer has the following structure:

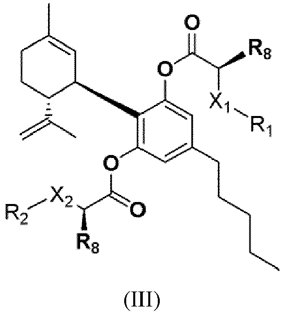

In some embodiments of the present invention, the stereoisomer described above has the following structure:

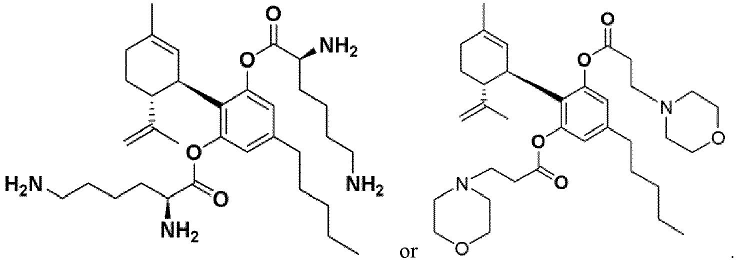

In a third aspect of the present invention, provided is a salt or cocrystal, particularly a pharmaceutically acceptable salt or cocrystal, of the compound according to the first aspect or the stereoisomer according to the second aspect. Further, the salt is a base addition salt or an acid addition salt. The base addition salt can be formed from a metal or an amine with the compound, particularly an alkali metal salt or an alkaline earth metal salt, e.g., a sodium salt, a potassium salt, a magnesium salt, or a calcium salt, particularly a sodium salt; the acid addition salt can be formed from an inorganic or organic acid with the compound, e.g., hydrochloride, nitrate, sulfate, phosphate, citrate, formate, fumarate, maleate, acetate, succinate, tartrate, mesylate, or p-toluenesulfonate, particularly methanesulfonate. In a fourth aspect of the present invention, provided is a prodrug, a solvate, or a metabolite of the compound according to the first aspect. In a fifth aspect of the present invention, provided is a preparation method for the compound according to the first aspect.

Further, the preparation method can comprise the following reaction step:

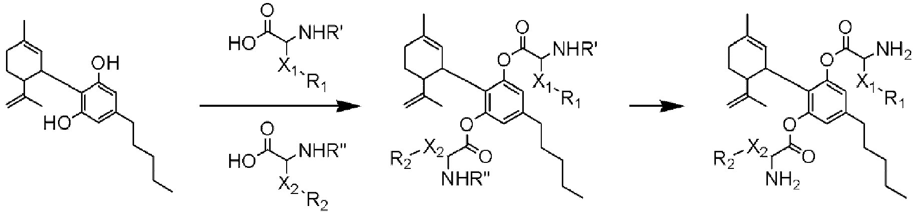

Further, R' and R" are amino protecting groups, e.g., alkoxycarbonyl (e.g., -Boc), acyl, or alkyl.

Further, the reaction described above is performed at 20-30 °C, e.g., room temperature.

In a sixth aspect of the present invention, provided is a pharmaceutical composition comprising the compound according to the first aspect of the present invention or the pharmaceutically acceptable salt, the cocrystal, the stereoisomer, the prodrug, the solvate or the metabolite thereof, and one or more pharmaceutically acceptable excipients.

Further, the pharmaceutically acceptable excipients can be selected from: one or more of fillers, binders, lubricants, disintegrants, antioxidants, buffers, suspending agents, solubilizers, thickeners, stabilizers, preservatives, and the like.

Further, the pharmaceutical composition can be administered enterally or parenterally, e.g., by intravenous, intramuscular, intradermal, and subcutaneous routes.

Further, the pharmaceutical composition can be prepared into the following dosage forms: tablets, pills, powders, granules, capsules, lozenges, syrups, gels, emulsions, suspensions, controlled release preparations, aerosols, powder inhalations, films, injections, intravenous drip infusions, transdermal absorption formulations, ointments, lotions, adhesive formulations, suppositories, nasal formulations, pulmonary formulations, and the like.

Further, various dosage forms of the pharmaceutical composition can be prepared according to conventional production methods in the pharmaceutical field.

Further, the pharmaceutical composition can also comprise one or more other active ingredients for the same or different indications.

In a seventh aspect of the present invention, provided is use of the compound according to the first aspect of the present invention, or the pharmaceutically acceptable salt, the cocrystal, the stereoisomer, the prodrug, the solvate or the metabolite thereof, and the pharmaceutical composition according to the sixth aspect of the present invention in the preparation of a medicament for preventing and/or treating a disease.

Further, the disease described above is selected from: one or more of a nervous system disease, cancer, an autoimmune disease, a cardiovascular disease, pain, inflammation, and liver injury, particularly a nervous system disease, and preferably, the disease is a nervous system disease.

Further, the nervous system disease is a neurodegenerative disease or epilepsy. The neurodegenerative disease is selected from: Parkinson's disease, Alzheimer's disease, Creutzfeldt-Jakob disease, Huntington's disease, and multiple sclerosis; preferably, the neurodegenerative disease is Parkinson's disease.

Further, the cancer includes, but is not limited to, breast cancer, lung cancer, colorectal cancer, liver cancer, pancreatic cancer, gastric cancer, gastroesophageal adenocarcinoma, esophageal cancer, small intestine cancer, gastric cardia cancer, endometrial cancer, ovarian cancer, fallopian tube cancer, vulval cancer, testicular cancer, prostate cancer, leukemia, glioma, and the like.

Further, the autoimmune disease includes, but is not limited to, systemic lupus erythematosus, rheumatoid arthritis, multiple sclerosis, ulcerative colitis, Crohn's disease, autoimmune hepatitis, and the like.

In an eighth aspect of the present invention, provided is a method for preventing and/or treating a disease, which comprises a step of administering to a subject in need thereof the compound according to the first aspect of the present invention, or the pharmaceutically acceptable salt, the cocrystal, the stereoisomer, the prodrug, the solvate or the metabolite thereof, or the pharmaceutical composition according to the sixth aspect.

Further, the disease described above is selected from: one or more of a nervous system disease, cancer, an autoimmune disease, a cardiovascular disease, pain, inflammation, and liver injury, particularly a nervous system disease, and preferably, the disease is a nervous system disease.

Further, the nervous system disease is a neurodegenerative disease or epilepsy. The neurodegenerative disease is selected from: Parkinson's disease, Alzheimer's disease, Creutzfeldt-Jakob disease, Huntington's disease, and multiple sclerosis; preferably, the neurodegenerative disease is Parkinson's disease.

Further, the cancer includes, but is not limited to, breast cancer, lung cancer, colorectal cancer, liver cancer, pancreatic cancer, gastric cancer, gastroesophageal adenocarcinoma, esophageal cancer, small intestine cancer, gastric cardia cancer, endometrial cancer, ovarian cancer, fallopian tube cancer, vulval cancer, testicular cancer, prostate cancer, leukemia, glioma, and the like.

Further, the autoimmune disease includes, but is not limited to, systemic lupus erythematosus, rheumatoid arthritis, multiple sclerosis, ulcerative colitis, Crohn's disease, autoimmune hepatitis, and the like.

Further, the subject may be a mammal, e.g., a human, chimpanzee, monkey, dog, rabbit, mouse, etc., particularly a human.

The inventor of the present invention carries out structural modification on the basis of cannabidiol, and obtains the compounds of the present invention by screening from a series of synthetic derivatives. Animal test results show that the compounds can reduce the balance beam score of the model animal of Parkinson's disease and improve dopamine level and tyrosine hydroxylase (TH) cell positive rate in substantia nigra (SubN), so that it can be used for drug development and research of various diseases such as Parkinson's disease, and has relatively good application value.

### BRIEF DESCRIPTION OF THE DRAWINGS

FIG. 1 shows results for the behavioral balance beam score of the model of Parkinson's disease.
FIG. 2 shows detection results for the content of dopamine in striatum of the model of Parkinson's disease after treatment.
FIG. 3 shows positive rate of tyrosine hydroxylase (TH) cells in substantia nigra (SubN).

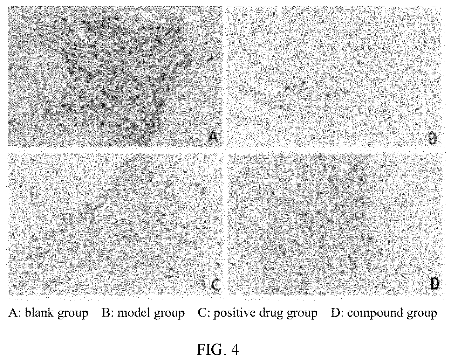

FIG. 4 shows micrographs of tyrosine hydroxylase (TH) cells in substantia nigra (SubN).

### DETAILED DESCRIPTION

Unless otherwise defined, all scientific and technical terms used in the present invention have the same meaning as commonly understood by those skilled in the art to which the present invention relates, for example:
The term "alkyl" refers to a hydrocarbyl group that is linear or branched and that does not contain unsaturated bonds, and that is linked to the rest of the molecule via a single bond. The alkyl used herein generally contains 1 to 12 (e.g., 1, 2, 3, 4, 5, 6, 7, 8, 9, 10, 11, or 12) carbon atoms, preferably 1 to 6 carbon atoms (i.e., C₁₋₆ alkyl).

Examples of the alkyl include, but are not limited to, methyl, ethyl, *n*-propyl, isopropyl, *n*-butyl, isobutyl, *tert-*butyl, *n*-pentyl, isopentyl, neopentyl, *tert*-pentyl, *n*-hexyl, isohexyl, and the like.

The term "alkylene" refers to hydrocarbyl (divalent alkyl) formed from an alkane molecule by losing two hydrogen atoms, which may be linear or branched and is linked to other parts of the molecule by a single bond. Typical alkylene herein contains 1 to 12 (e.g., 1, 2, 3, 4, 5, 6, 7, 8, 9, 10, 11, or 12) carbon atoms, preferably 1 to 6 carbon atoms. Examples of the alkylene include methylene (-CH₂-), ethylidene, propylidene, butylidene, and the like. The term "alkoxy" refers to a substituent formed from a hydroxy group by substituting the hydrogen atom with alkyl. The alkoxy used herein generally contains 1 to 12 (e.g., 1, 2, 3, 4, 5, 6, 7, 8, 9, 10, 11, or 12) carbon atoms, preferably 1 to 6 carbon atoms (i.e., C₁₋₆ alkoxy). Examples of the alkoxy include, but are not limited to, methoxy, ethoxy, propoxy, butoxy, and the like.

The term "cycloalkyl" refers to an alicyclic hydrocarbon, such as those containing 1 to 4 monocyclic and/or fused rings and having 3 to 18 carbon atoms, preferably 3 to 10 (e.g., 3, 4, 5, 6, 7, 8, 9, or 10) carbon atoms, e.g., cyclopropyl, cyclobutyl, cyclopentyl, cyclohexyl, adamantyl, or the like.

The term "aryl" refers to any functional group or substituent derived from a simple aromatic ring, including monocyclic aryl groups and/or fused aryl groups, such as those containing 1 to 3 monocyclic or fused rings, and having 6 to 18 (e.g., 6, 8, 10, 12, 14, 16, or 18) carbon ring atoms. The aryl used herein is generally an aryl group containing 1 to 2 monocyclic or fused rings and having 6-12 carbon ring atoms (i.e., C₆₋₁₂ aryl), wherein H on the carbon atoms may be substituted, for example, with alkyl, halogen, and the like. Examples of the aryl include, but are not limited to, phenyl, *p*-hydroxyphenyl, and the like.

The term "pharmaceutically acceptable salt" includes both acid addition salts and base addition salts.

The term "cocrystal" refers to a crystal formed by combining the compound of the present invention with other physiologically acceptable acids, bases, or non-ionic compounds through hydrogen bonding, van der Waals force, π-π stacking, halogen bonding and other non-covalent bonding effects.

The term "stereoisomer" includes enantiomeric, diastereomeric, and geometric isomer forms.

The term "solvate" refers to a physical association of the compound of the present invention with one or more solvent molecules. The physical association includes various degrees of ionic and covalent bonding, including hydrogen bonding. In some cases, the solvate can be isolated, for example, when one or more solvent molecules are incorporated into the crystal lattice of a crystalline solid. Solvates include both solution phases and isolatable solvates. Representative solvates include ethanolates, methanolates, and the like.

The term "metabolite" refers to a product resulting from chemical degradation of the compound of the present invention under physiological conditions *in vivo.*

The term "prodrug" refers to forms of the compound of formula I (including acetals, esters, and zwitterions) which are suitable for administration to patients without undue toxicity, irritation, allergic response and the like, and which are effective for the intended use thereof. The prodrug is converted *in vivo,* e.g., by hydrolysis in blood, to give the parent compound.

In the present invention, "D" represents deuterium; "deuterated" means that one or more hydrogen atoms are substituted with the corresponding number of deuterium atoms.

The term "subject" refers to any animal or cell thereof, whether *in vitro* or *in situ,* that receives the methods described herein. Further, the animal described above includes mammals, e.g., rats, mice, guinea pigs, rabbits, dogs, monkeys, chimpanzees, or humans, particularly humans.

The term "treating" refers to preventing, curing, reversing, attenuating, alleviating, minimizing, suppressing, arresting, and/or stopping one or more clinical symptoms of a disease after its onset.

The term "preventing" refers to treatment to avoid, minimize, or make difficult the onset or progression of a disease prior to its onset.

The disclosures of the various publications, patents, and published patent specifications cited herein are hereby incorporated by reference in their entireties.

The technical solutions of the present invention will be clearly and completely described below with reference to the examples of the present invention, and it is obvious that the described examples are only a part of the examples of the present invention but not all of them. Based on the examples of the present invention, all other examples obtained by those of ordinary skills in the art without creative work shall fall within the protection scope of the present invention.

### Example 1: Preparation of Compound 3

### 1. Synthetic route

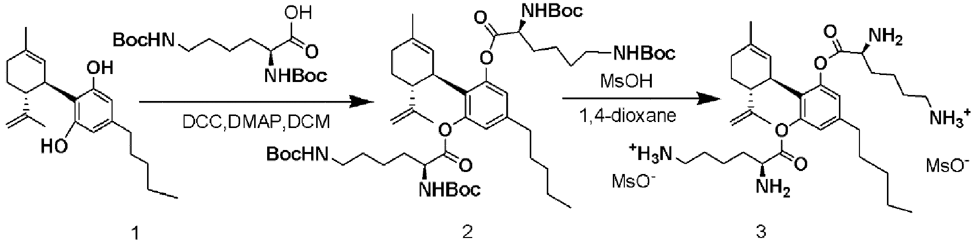

### 2. Synthetic method

### (1) Preparation of compound 2

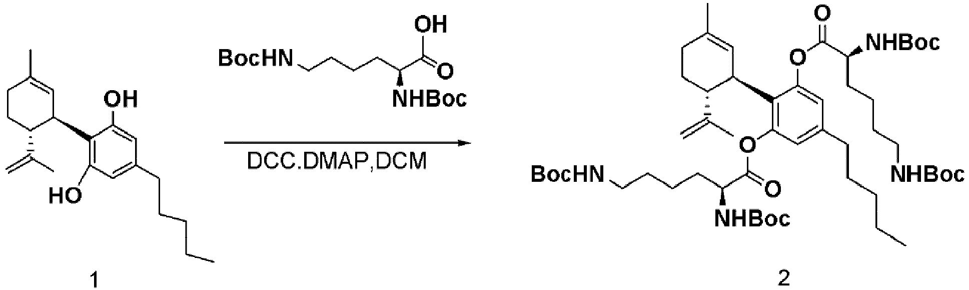

To a 1000 mL single-necked flask were added compound 1 (CBD, 10 g, 31.85 mmol) and dichloromethane (500 mL), and the mixture was stirred for dissolution. Subsequently, *N*α,*N*ε-bis(Boc)-L-lysine (23.14 g, 66.88 mmol), dicyclohexylcarbodiimide (13.78 g, 66.88 mmol), and 4-dimethylaminopyridine (8.16 g, 66.88 mmol) were added, and the mixture was stirred at room temperature overnight. Insoluble substances generated in the reaction were filtered out, and the filtrate was washed with saturated brine. The organic phase was dried over anhydrous sodium sulfate, filtered, and concentrated under reduced pressure to give a crude product (41 g) as an oil. The crude product was subjected to silica gel column chromatography (n-hexane:ethyl acetate = 5:1-3:1) to give a solid compound 2 (13.5 g) as a foam. MS (ESI): 988 [M+NH₄]⁺.

### (2) Preparation of compound 3

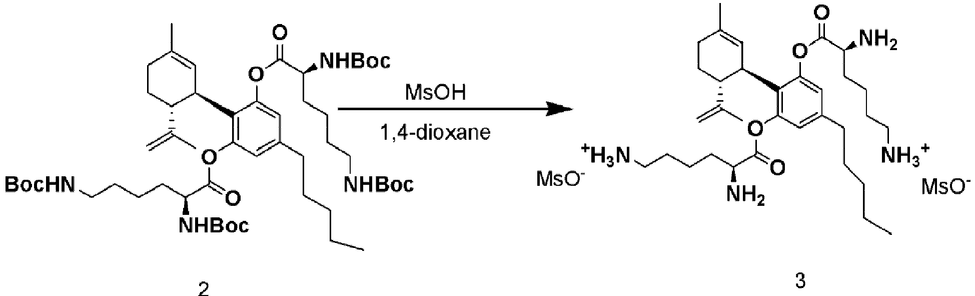

To a 250 mL single-necked flask were added compound 2 (5.7 g, 5.88 mmol) and 1,4-dioxane (60 mL), and the mixture was stirred for dissolution. Subsequently, methanesulfonic acid (3.8 mL, 58.8 mmol) was added to the reaction flask under nitrogen atmosphere, and after the addition, the mixture was stirred at room temperature overnight. A viscous solid was generated in the reaction flask. A clear liquid was poured out, and the residual solid was dried in vacuum to give a crude product (6.8 g). The crude product was purified by C18 column chromatography (acetonitrile:water = 2:100-4:100). The eluate was detected by HPLC, collected, and lyophilized to give compound 3 (2.5 g, 92% purity) as a white foamy solid. MS (ESI): 286 [(M+2H)/2]⁺.

### Example 2: Study on Treatment for Parkinson's Disease

### 1. Laboratory animals and some reagents

C57BL6 mice, SPF grade, weighing 18-20 g, aged 6-8 weeks, male, purchased from Shanghai Slac Laboratory Animal Co., Ltd. Feeding environment: the temperature was kept at 22 ± 1 °C, and the humidity was kept at 65%-70%, in a 12/12 hour light/dark illumination cycle, with free access to water.

MPTP, purchased from Sigma; amantadine, purchased from Sigma; and absolute ethanol, purchased from Sinopharm Chemical Reagent Co., Ltd.

### 2. Experimental grouping

Laboratory animals were randomly divided into 4 groups: blank group, model group, positive drug group, and compound group, with 8 animals in each group.

### 3. Experimental procedures

(1) 32 SPF-grade C57 mice aged 6-8 weeks were purchased and acclimatized for one week, and subsequently the modeling was started.
(2) MPTP (25 mg/kg) was intraperitoneally injected once a day for 7 consecutive days. The blank group was left untreated.
(3) After modeling, administration was started. The positive drug group was subjected to intragastric administration of amantadine (40 mg/kg), and the compound group was subjected to intragastric administration of compound 3 (prepared in Example 1) at a determined dose (100 mg/kg). The intragastric administration was performed once a day for 14 consecutive days.
(4) Behavioral test was first performed by balance beam test scoring.

A beam of 80 cm long and 2.5 cm wide was horizontally fixed at a height of 10 cm from the table top, and subsequently, the animals were allowed to walk on the beam.

The scoring scale was as follows: being able to jump on the balance beam and to walk freely without falling down: 0 points; being able to jump on the balance beam, but having a probability of tumbling down of less than 50% when walking on the balance beam: 1 point; being able to jump on the balance beam, but having a probability of tumbling down of greater than 50% when walking on the balance beam: 2 points; being able to jump on the balancing beam with the help of the normal side of the body, but failing to get help from the hind limb on the paralyzed side to move forward: 3 points; being unable to walk on the balance beam, but being able to sit on the beam: 4 points; falling off quickly when being placed on the beam: 5 points.

### (5) After the scoring, the sampling was performed.

After the mice were sacrificed quickly, the brains of the mice were taken and the cerebral palliums (CPs) of the mice were isolated. After exposing the brain striatum of the mice, the striatum was weighed and homogenized after adding PBS according to a proportion. The supernatant was isolated and detected for the content of dopamine in the brain striatum.

### 4. Determination of content of dopamine in brain striatum by the kit method

### 4.1 The kits and some instruments used in the experiment are shown in the table below.

**Table 1. Kit information**

| Antibody name | Source of antibody | Catalog No. |
|---|---|---|
| Mouse dopamine (DA) detection kit | NanJing JianCheng | H170 |
| Protein quantification (BCA method) test kit | NanJing JianCheng | A045-3 |

**Table 2. Main instruments and consumables**

| Instrument name | Manufacturer | Model |
|---|---|---|
| Microplate reader | Finland (Labsystems Multiskan MS) corporation | Model 352 |
| Microplate washer | Finland (Thermo Labsystems) corporation | AC8 |
| Centrifuge | High speed microcentrifuge (domestic) | TG16W |
| Thermostatic incubator | Insulated thermostatic incubator (domestic) | Model GNP-9080 |
| Pipettor | P-type pipettor (Gilson Company, Inc.) | P2, P10, P20, P100, P200, P1000 |

### 4.2 Protein quantification

(1) Standard curve plotting: a microplate was taken, and reagents were added according to the table below.

**Table 3. Reagents**

| Well number | 1 | 2 | 3 | 4 | 5 | 6 | 7 | 8 |
|---|---|---|---|---|---|---|---|---|
| Protein standard solution (µL) | 0 | 2 | 4 | 6 | 8 | 12 | 16 | 20 |
| Deionized water (µL) | 20 | 18 | 16 | 14 | 12 | 8 | 4 | 0 |
| Protein concentration (µg/µL) | 0 | 0.05 | 0.1 | 0.15 | 0.2 | 0.3 | 0.4 | 0.5 |

(2) According to the number of samples, a proper amount of BCA working solution was prepared by uniformly mixing BCA reagent A and reagent B according to a volume ratio of 50:1.
(3) 160 µL of BCA working solution was added to each well.
(4) The microplate was shaken on a shaker for 30 s, left to stand at 37 °C for 30 min, and subsequently measured for absorbance at 562 nm. A standard curve was plotted with the absorbance value as an abscissa and the protein concentration (µg/µL) as an ordinate.
(5) 2 µL of test protein and 18 µL of PBS (10-fold dilution) were added to the microplate, 160 µL of BCA working solution was added, and the microplate was shaken on a shaker for 30 s, left to stand at 37 °C for 30 min, and subsequently measured for the absorbance at 562 nm.
(6) According to the absorbance value of the test sample, the corresponding protein concentration (µg/µL) could be found on the standard curve, and the protein concentration was multiplied by a sample dilution factor (10) to obtain the actual concentration (µg/µL) of the sample.
(7) The experimental procedures and methods for the determination of dopamine in mice were referred to the kit instructions.

### 5. Determination of tyrosine hydroxylase (TH) cells in substantia nigra (SubN) by immunohistochemistry

### 5.1 Experimental materials

**Table 4. Main reagents**

| Reagent name | Manufacturer | Catalog No. |
|---|---|---|
| Paraffin | Sinopharm Chemical Reagent Co., Ltd. | 69018961 |
| Formaldehyde | Sinopharm Chemical Reagent Co., Ltd. | 10010018 |
| Xylene | Sinopharm Chemical Reagent Co., Ltd. | 10023418 |
| Absolute ethanol | Sinopharm Chemical Reagent Co., Ltd. | 10092680 |
| 30%H2O2 | Sinopharm Chemical Reagent Co., Ltd. | 10011218 |
| Broad-spectrum secondary antibody | Shanghai Long Island Biotec. Co., Ltd. | D-3004 |
| DAB concentrated kit | Shanghai Long Island Biotec. Co., Ltd. | FL-6001 |
| Hematoxylin | BASO | 714094 |
| Neutral resin | Beijing Solarbio | G8590 |
| Anti-TyrosineHydroxylase | abcam | ab137869 |

**Table 5. Main instruments and consumables**

| Instrument name | Manufacturer | Model |
|---|---|---|
| Upright microscope | NIKON | ECLIPSE Ni |
| Pipettor | P-type pipettor (Gilson Company, Inc.) | P2, P10, P20, P100, P200, P1000 |
| Constant temperature oven | Shanghai Yiheng Technology Instrument Co., Ltd. | DHG-9023A |
| Paraffin slicing machine | Leica | SQ2125 |
| Slice spreading machine | Leica | PPTHK-21B |
| Micrograph analysis system | NIKON | DS-Ri2 |

### Preparation of solutions

### (1) PBS solution

8 g of NaCl, 0.2 g of KCl, 1.44 g of Na₂HPO₄, and 0.24 g of KH₂PO₄ were dissolved in 600 mL of ddH₂O. The solution was adjusted to pH 7.4 with HCl, and after the volume was made up to 1 L, the solution was filtered through a filter, autoclaved, and stored at room temperature.

### (2) 0.1 mol/L sodium citrate buffer

3.8 mL of 0.1 mol/L citric acid and 16.2 mL of 0.1 mol/L sodium citrate.

Citric acid C₆H₈O₇·H₂O: molecular weight: 210.14, 21.01 g/L for a 0.1 mol/L solution.

Sodium citrate Na₃C₆H₅O₇·2H₂O: molecular weight: 294.12, 29.41 g/mL for a 0.1 mol/L solution.

### 5.2 Experimental procedures

### 5.2.1 Sample embedding and fixing

### (1) Sampling and fixing

The tissue should be cut using sharp knives and scissors. When cutting a tissue mass, the tissue was cut by pulling backwards from the root of the knife. The tissue mass had a thickness of about 0.2-0.3 cm and a size of 1.5 cm × 1.5 cm × 0.3 cm. The prepared tissue mass was fixed in 10% formalin for 48 h.

### (2) Washing and dehydration

The fixed tissue was washed with running water to remove residual fixing liquid and impurities. The tissue mass was dehydrated in stages with different concentrations of ethanol (50%, 70%, 85%, 95%, up to pure alcohol (absolute ethanol)), each stage for 2 h. Dehydration must be performed in a capped flask to prevent high concentrations of ethanol from absorbing moisture in the air, resulting in reduced concentrations and incomplete dehydration. The material to be preserved can remain in 70% ethanol when being dehydrated with it. For long-term preservation, an equivalent amount of glycerol can be added.

### (3) Transparentizing

The tissue mass was placed into a mixed solution of pure ethanol and xylene in an equivalent volume and left to stand for 2 h, subsequently placed into pure xylene and left to stand for two hours, and placed into pure xylene and left to stand for another two hours. After transparentizing, the effect of the previous dehydration of the material can be shown. If the dehydration is complete, the tissue is in a transparent state, and if there is a white cloud-shaped substance in the tissue, it means that the dehydration is incomplete and must be reworked, but the effect of rework is often not good. When xylene is used for transparentizing, it should be prevented from volatilizing and absorbing moisture in the air, and it should be kept in an anhydrous state.

### (4) Paraffin immersion

The paraffin immersion must be performed in a thermostat. The tissue mass material was first immersed in a mixed solution of molten paraffin and xylene in equal volume for 1-2 h and subsequently immersed in 2 parts of molten paraffin solutions for 3 h each.

### (5) Embedding

Embedding is the encapsulation of the paraffin-impregnated tissue mass with paraffin. Specifically, a paper box was first prepared, molten paraffin was added to the box, a tissue mass was quickly clamped by using a pair of pre-heated tweezers, and placed flat at the bottom of the paper box with a cut surface facing downwards. Subsequently, the paper box was slightly lifted up and placed flat in cold water, immediately pressed into the water after the paraffin on the surface was solidified for quickly cooling and solidifying the paraffin, and taken out after 30 min.

### (6) Slicing

The paraffin block was placed in a refrigerator at -20 °C for at least 30 min before slicing to increase hardness. The slicing knife was arranged on a knife rest of the slicing machine and was tightly fixed, a paraffin block base or a paraffin block was fixed, and the paraffin block and the knife were adjusted to proper positions, with the knife edge at an angle of 5 degrees to the surface of the paraffin block. The thickness for slicing on the slicing machine was adjusted to 4-7 µm before the slicing was performed.

### 5.2.2 Immunohistochemical staining

### (1) Slice baking and deparaffinating

A slide was baked in a constant temperature oven at 65 °C for slice baking for 30 min, soaked in xylene I for 15 min, and subsequently in xylene II for 15 min.

### (2) Hydration

The deparaffinated slice was sequentially soaked in 100% alcohol, 95% alcohol, 85% alcohol, and 75% alcohol for 5 min, and washed with tap water for 10 min.

### (3) Antigen retrieval

The slice was retrieved at high pressure in 0.01 M sodium citrate buffer for 15 min, and subsequently washed with 0.02 M PBS 3 times for 3 min each after natural cooling.

### (4) Blocking

The slide was placed in 3% H₂O₂ and incubated in a wet box for 10 min to eliminate the activity of endogenous peroxidase. The slide was washed with 0.02 M PBS 3 times for 3 min each.

### (5) Incubation with primary antibody

A primary antibody (1:2000 dilution) was added dropwise, and the slide was incubated in a wet box, and left to stand at room temperature for 1 h (or incubated at 4 °C overnight). The slide was washed with 0.02 M PBS 3 times for 3 min each.

### (6) Incubation with secondary antibody

HRP was added to label the broad-spectrum secondary antibody, and the slide was incubated in a wet box, and left to stand at room temperature for 20-30 min. The slide was washed with 0.02 M PBS 3 times for 3 min each.

### (7) Color development

The slice was stained with DAB, and when a color change was observed in the slice, the staining liquid was immediately washed away with tap water.

### (8) Hematoxylin staining

The slice was re-stained with hematoxylin for 3 min, differentiated with 1% hydrochloric acid alcohol, and observed under a microscope for controlling the staining degree. The slice was washed with tap water for 10 min and placed in an oven at 65 ° C for removing water by drying.

### (9) Transparentizing and sealing

The slide was placed in xylene for transparentizing 2 times for 3 min each, sealed with a neutral resin, and placed in an oven at 65 ° C for 15 min.

### 5.2.3 Image acquisition and analysis

The slice was photographed through the microscope, and the relevant parts of the sample were collected and analyzed to calculate the positive area.

### 6. Experimental results

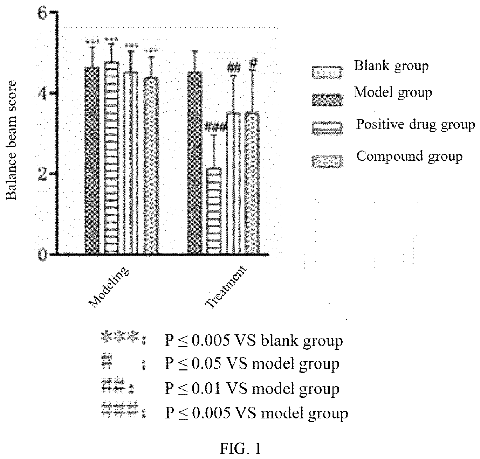

(1) The results for the behavioral balance beam score of the model of Parkinson's disease are shown in the table below and in FIG. 1.

**Table 6. Results for behavioral balance beam score of the model of Parkinson's disease**

| Group | After modeling | After treatment |
|---|---|---|
| Blank group | 0 | 0 |
| Model group | 4.63±0.52*** | 4.50±0.53 |
| Positive drug group | 4.75±0.46*** | 2.13±0.83### |
| Compound group (compound 3 prepared in Example 1) | 4.38±0.52*** | 3.50±1.07# |

The mice were intraperitoneally injected with MPTP for the preparation of the model of Parkinson's disease, and subjected to behavioral evaluation by balance beam scoring. The balance beam scoring was performed 7 days after intraperitoneal injection of MPTP. Except for the blank group, which was not modeled and had a balance beam score of 0 points, all other groups of mice had scores of 5-6 points, with P ≤ 0.05 for the comparison of the other groups with the blank group, which was statistically significant, proving that the modeling was successful.

After subsequent treatment with the positive drug and the compound group, the model group had a balance beam score of 4-5 points and the positive drug group had a balance beam score of 2-3 points. The compound group had a balance beam score of 3-4 points. The positive drug group and the compound group showed a significant descending trend as compared to the model group, with P ≤ 0.05, which was statistically significant.

After treatment, the groups were ranked in terms of the scores as follows: blank group < positive drug group < compound group < model group.

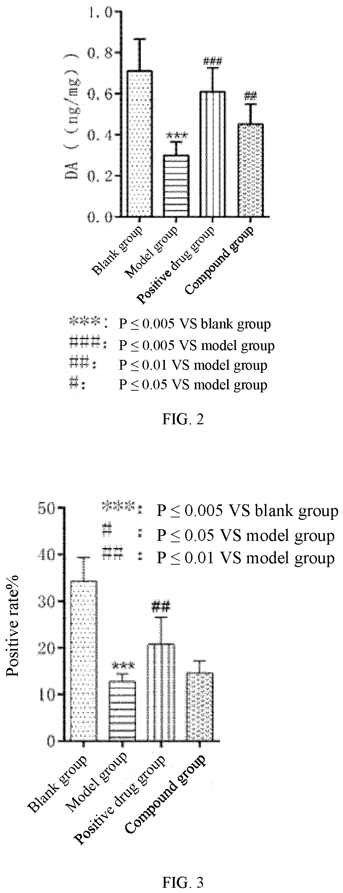

(2) The content of dopamine in striatum of the model of Parkinson's disease after treatment is shown in the table below and in FIG. 2.

**Table 7. Content of dopamine in striatum of the model of Parkinson's disease after treatment**

| Group | DA (ng/mg) |
|---|---|
| Blank group | 0.71±0.16 |
| Model group | 0.30±0.07*** |
| Positive drug group | 0.61±0.12### |
| Compound group (compound 3 prepared in Example 1) | 0.45±0.10## |

Dopamine is used as neurotransmitter to regulate various physiological functions of the central nervous system. The effect of drug treatment of the model of Parkinson's disease was determined by determining the content of dopamine in striatum of the model of Parkinson's disease of treated mice.

The degree of decrease in the content of dopamine in the model was determined by comparing the content of dopamine in the groups with that in the blank group. Except for the positive drug group, the compound group showed a significant decrease in the content of dopamine, with P ≤ 0.05, which was statistically significant.

Subsequently, through the comparison of the dopamine content in the compound group with that in the model group, the group showed an increase in the dopamine content as compared to the model group, with P ≤ 0.01, which was statistically significant, indicating that the compound group had the function of increasing the dopamine content in the brain.

The groups were ranked in terms of the content of dopamine in striatum as follows: blank group > positive drug group > compound group > model group.

(3) The experimental results for the determination of tyrosine hydroxylase (TH) cells in substantia nigra (SubN) by immunohistochemistry are shown in the table below and in FIG.s 3 and 4.

**Table 8. Experimental results for determination of tyrosine hydroxylase (TH) cells in substantia nigra (SubN) by immunohistochemistry**

| Group | TH cell positive rate (%) |
|---|---|
| Blank group | 34.27±5.08 |
| Model group | 12.74±1.71*** |
| Positive drug group | 20.82±5.77## |
| Compound group (compound 3 prepared in Example 1) | 14.59±2.60 |

Tyrosine hydroxylase (TH) is a monooxygenase, which is a rate-limiting enzyme in the first step of a series of reactions in which organisms are catalyzed to synthesize L-dopamine (DA) themselves, and is expressed only in the cytoplasm. TH is abundant in neurons. A neuron which is positive for the TH immune response of the midbrain is a DA neuron due to the lack of dopamine-β-hydroxylase in the midbrain, which can be used as a marker of dopaminergic neurons in the brain. In the body, L-tyrosine is used to generate levodopa under the catalysis of tyrosine hydroxylase, and levodopa is subjected to carboxyl removal under the catalysis of aromatic decarboxylase to finally generate L-dopamine. Due to the important role of tyrosine hydroxylase in dopamine synthesis, its function loss or underexpression directly affects the synthesis and secretion of dopamine. Dopamine is an important neurotransmitter, and the inability to synthesize or insufficient secretion of dopamine by dopaminergic neurons leads to Parkinson's disease.

The experimental results showed that in the mouse model of Parkinson's disease established by the intraperitoneal injection of MPTP, the positive rate of tyrosine hydroxylase (TH) cells in substantia nigra (SubN) of the mice was decreased, with P ≤ 0.05, which was statistically significant. The positive drug group showed a significant increase in the positive rate of tyrosine hydroxylase (TH) cells in substantia nigra (SubN) as compared to the model group, indicating that the modeling was successful.

Through the comparison of the treatment effect on the mouse model of Parkinson's disease of the compound group with that of the model group, the compound group showed an increase in the positive rate of tyrosine hydroxylase (TH) cells in substantia nigra (SubN) as compared to the model group, with P ≤ 0.01 for the comparison of the positive rate of tyrosine hydroxylase (TH) cells in substantia nigra (SubN) of the compound group with that of the model group, which was statistically significant.

Through the comparison of the positive rates of tyrosine hydroxylase (TH) cells in substantia nigra (SubN) of the groups after treatment, the groups were ranked as follows: blank group > positive drug group > compound group > model group.

### 7. Summary

In the experiment, the mouse model of Parkinson's disease was established by the intraperitoneal injection of MPTP (25 mg/kg) once a day for 7 consecutive days, and was screened by the balance beam scoring.

Subsequently, the mice were subjected to treatment by intragastric administration of the positive drug and the compound. After the treatment by intragastric administration for 14 consecutive days, the differences between the compound group and the blank and model groups were identified by analyzing the balance beam scores of the groups after treatment, determining the content of dopamine in striatum by ELISA, determining the positive rate of tyrosine hydroxylase (TH) cells in substantia nigra (SubN) by immunohistochemistry, and other assay methods, and subsequently statistical analysis was performed to analyze the effectiveness of compound 3.

Through the evaluation of the balance beam score after treatment, the positive drug group and the compound group showed a significant descending trend as compared to the model group, with P ≤ 0.05, which was statistically significant.

Subsequently, through the comparison of the content of dopamine in the compound group with that in the model group, the compound group showed an increase in the content of dopamine as compared to the model group, with P ≤ 0.01, which was statistically significant.

Through the comparison of the positive rates of the positive drug group and the compound group with that of the model group after the treatment of the mouse model of Parkinson's disease with the compound and the positive drug, it was shown that P ≤ 0.01 for the comparison of the positive rate of tyrosine hydroxylase (TH) cells in substantia nigra (SubN) of the compound group with that of the model group, which was statistically significant. Through overall analysis of the comparison of the compound group with the model group, the compound group had a certain therapeutic effect, and compound 3 had a better therapeutic effect through the comparison of detection indexes.

The above description is only for the purpose of illustrating the preferred examples of the present invention, and is not intended to limit the scope of the present invention. Any modifications, equivalents, and the like made without departing from the spirit and principle of the present invention shall fall in the protection scope of the present invention.

The foregoing examples and methods described in the present invention may vary based on the abilities, experience, and preferences of those skilled in the art.

The certain order in which the steps of the method are listed in the present invention does not constitute any limitation on the order of the steps of the method.

## Claims

1. A compound, or a pharmaceutically acceptable salt, a cocrystal, a stereoisomer, a prodrug, a solvate or a metabolite thereof, wherein the compound has the following structure: 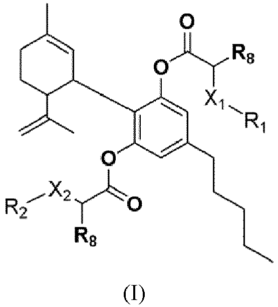 wherein,
R₈ is selected from: -NH₂ and -H;
X₁ and X₂ are independently selected from: a single bond, alkylene substituted with one or more substituents, alkylene spaced by one or more -C(=O)O-, alkylene spaced by one or more -OC(=O)-, alkylene spaced by one or more alkylimino, alkylene spaced by one or more -OC(=O)O-, and alkylene spaced by a combination of the above spacer groups; the substituent is selected from: alkyl, hydroxy, halogen, aryl, cycloalkyl, thioalkoxy, nitro, cyano, amino, heteroaryl, and heterocycloalkyl, wherein H on the C atoms can be substituted with halogen, -OC₀₋₁₀ alkyl, linear/branched C₁₋₁₀ alkyl, nitro, cyano, or amino;
R₁ and R₂ are independently selected from: -H, 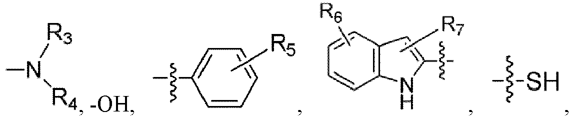 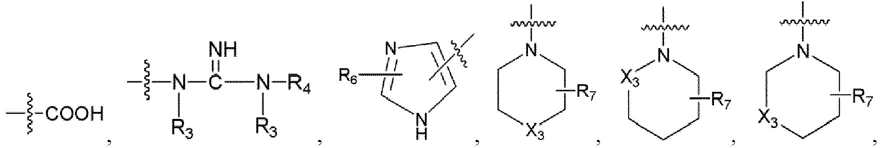 and alkoxy, wherein, X₃ is selected from N, O, and S; preferably, R₁ and R₂ are independently selected from: 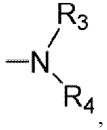 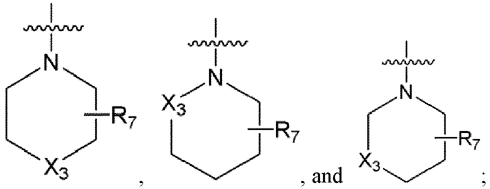
R₃ and R₄ are each independently selected from: -H, halogen, alkyl, cycloalkyl, aryl, heteroaryl, and heterocycloalkyl;
R₅, R₆, and R₇ are each independently selected from: -H, -OH, halogen, -NOz, -CN, alkyl, and cycloalkyl;
or general formula (I) is optionally substituted with 1 or more D atoms.

2. The compound according to claim 1, wherein the stereoisomer has the following structure:

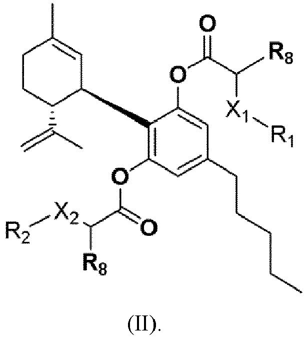

3. The compound according to claim 1, wherein R₈ is -NH₂; X₁ and X₂ are independently selected from: C₁₋₅ alkylene and alkylene substituted with one or more substituents selected from: alkyl, hydroxy, halogen, aryl, cycloalkyl, thioalkoxy, nitro, cyano, amino, heteroaryl, and heterocycloalkyl, wherein H on the C atoms can be substituted with halogen, -OC₀₋₁₀ alkyl, linear/branched C₁₋₁₀ alkyl, nitro, cyano, and amino; R₃ and R₄ are both - H; R₅ is selected from: -H or -OH; R₆ and R₇ are both -H; R₁ and R₂ are independently selected from: -NH₂, - OH, 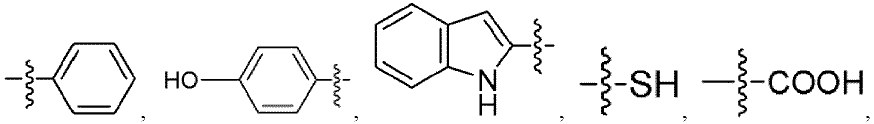 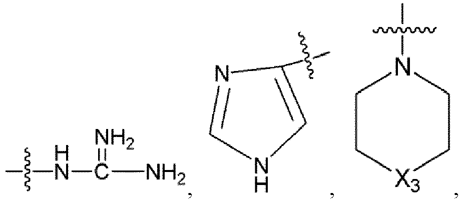 and alkoxy, wherein, X₃ is selected from N, O, and S, preferably X₃ is O.

4. The compound according to claim 1, wherein R₁ and R₂ are independently selected from: -NH₂ and

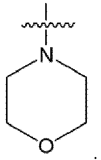

5. The compound according to claim 1, wherein R₁ and R₂ are both -NH₂.

6. The compound according to claim 1, wherein X₁ and X₂ are selected from: -CH₂-, -CH₂CH₂-, -(CH₂)₂CH₂-, - (CH₂)₃CH₂-, -CH₂CH(CH₃)₂-, -(CH₂)₄CH₂-, -CH(CH₃)CH₂CH₂-, and -CH(OH)CH₂-.

7. The compound according to claim 1, wherein X₁ and X₂ are both -(CH₂)₃CH₂-.

8. The compound according to claim 1, wherein the compound has the following structure:

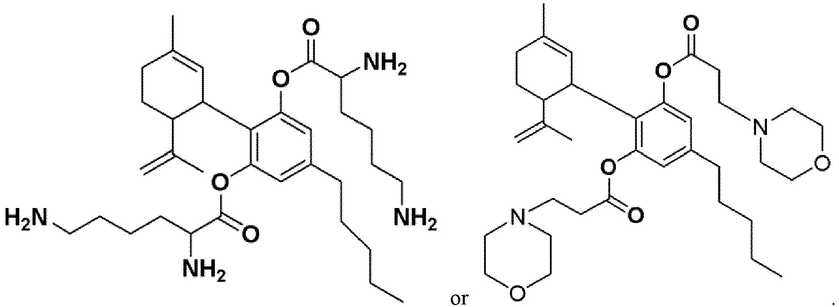

9. A pharmaceutical composition comprising the compound according to any one of claims 1-8, or the pharmaceutically acceptable salt, the cocrystal, the stereoisomer, the prodrug, the solvate or the metabolite thereof, and one or more pharmaceutically acceptable excipients.

10. Use of the compound according to any one of claims 1-7, or the pharmaceutically acceptable salt, the cocrystal, the stereoisomer, the prodrug, the solvate or the metabolite thereof, in the preparation of a medicament for preventing and/or treating a disease, wherein, the disease is selected from: one or more of a nervous system disease, cancer, an autoimmune disease, a cardiovascular disease, pain, inflammation, and liver injury; preferably, the disease is a nervous system disease.

11. The use according to claim 10, wherein the nervous system disease is neurodegenerative disease or epilepsy, wherein the neurodegenerative disease is selected from: Parkinson's disease, Alzheimer's disease, Creutzfeldt-Jakob disease, Huntington's disease, and multiple sclerosis; preferably, the neurodegenerative disease is Parkinson's disease;
the cancer is selected from: breast cancer, lung cancer, colorectal cancer, liver cancer, pancreatic cancer, gastric cancer, gastroesophageal adenocarcinoma, esophageal cancer, small intestine cancer, gastric cardia cancer, endometrial cancer, ovarian cancer, fallopian tube cancer, vulval cancer, testicular cancer, prostate cancer, leukemia, and glioma;
the autoimmune disease is selected from: systemic lupus erythematosus, rheumatoid arthritis, multiple sclerosis, ulcerative colitis, Crohn's disease, and autoimmune hepatitis.
